# EUROPEAN PATENT APPLICATION

(11) **EP 4 733 323 A1**
(43) Date of publication of application: **29.04.2026**
(21) Application number: 24847564.2
(22) Date of filing: 06.03.2024
(51) Int. Cl.: C07K 16/28, C12N 15/13, A61K 39/395, A61P 35/00, G01N 33/68

(54) **ANTIBODY OR ANTIGEN-BINDING FRAGMENT THEREOF AND USE THEREOF**

(30) Priority: 03.08.2023 CN 202310972000
(71) Applicant: SYNVAC BIOSCIENCES (XI'AN) CO., LTD., Shaanxi 710075 (CN)
(72) Inventor: LIAO, Xuebin, Xi'an, Shaanxi 710075 (CN); SI, Jingwen, Xi'an, Shaanxi 710075 (CN); GONG, Mengmeng, Xi'an, Shaanxi 710075 (CN)
(74) Representative: HGF
(86) International application number: PCT/CN2024/080370
(87) International publication number: WO 2025/025597

(57) **Abstract**

Provided are an antibody or an antigen-binding fragment thereof and a use thereof. The antibody or the antigen-binding fragment thereof specifically binds to IL13Rα2 and does not cross-bind to IL13Rα1, exhibits a significant killing effect on tumor cells expressing IL13Rα2, and can promote the release of cytokines (particularly IFN-γ and IL-2).

## Description

### TECHNICAL FIELD

The present disclosure relates to the technical field of immunology and molecular biology, and in particular to an antibody or an antigen-binding fragment thereof and use thereof.

### BACKGROUND

IL13Rα2 is a glycosylated protein with a molecular weight of about 56 kDa. Human IL13Rα2 comprises a signal sequence of 26 amino acids and an intracellular region of 17 amino acids. The short intracellular protein contains an unknown signal sequence. *In vitro,* IL13Rα2 binds to IL-13 with high affinity. Human IL13Rα2 features a similar amino-terminal fibronectin region, 4 conserved cysteine residues, and a conserved WSXWS motif in the extracellular domain. The N-linked glycosylated protein must bind to IL-13 intracellularly.

Studies have shown that IL13Rα2 is associated with various diseases. For example:
Non-patent literature: "The Functional Analysis of IL-13Rα1 and IL-13Rα2 in Atopic Dermatitis" (Xiao Song, Henan University, 2021) discloses that the expression level of IL13Rα2 is not high in healthy human skin, while it is significantly up-regulated in the skin of patients with atopic dermatitis. This up-regulated expression of IL13Rα2 is further verified in the skin of a mouse model of atopic dermatitis.

Non-patent literature: "Progress in Research on IL-13Rα2 in Tumors" (He Xiaoyan, Xiong Lixia, Journal of Nanchang University (Medical Science), 2013, 53(01):87-89) discloses that IL13Rα2 is expressed in various malignant tumors such as glioblastoma, brain tumors, ovarian cancer, liver cancer, malignant mesothelioma, renal cell carcinoma, thyroid cancer, epidermoid carcinoma, AIDS-related Kaposi's sarcoma, prostate cancer, and pancreatic cancer.

Non-patent literature: "Research on Striped Bamboo Shark Anti-Human IL-13Rα2 Single Domain Antibody" (Qin Lanyi, Zhejiang Sci-Tech University, 2021) discloses that IL13Rα2 is closely related to the occurrence and development of glioma, and is specifically highly expressed in brain glioma. Furthermore, it also discloses that the expression level of IL13Rα2 in the lung tissue of patients with idiopathic pulmonary fibrosis is significantly higher than that in normal lung tissue; lack of IL13Rα2 moderately protects against dextran sulfate sodium (DSS)-induced colitis; high expression of IL13Rα2 has also been confirmed on the surface of colorectal cancer cells; IL13Rα2 is also found to be an important marker of breast cancer, and its up-regulation is a cause of breast cancer invasion; IL13Rα2 is also highly expressed on the surface of renal cell carcinoma and clear cell renal cell carcinoma, and besides enhancing the invasion and migration capabilities of cancer cells, studies show that its high expression in clear cell renal cell carcinoma is also associated with the development of the resistance of cancer cells to sunitinib, a standard therapeutic drug.

Patent literature (CN116194481A) discloses that IL13Rα2 is overexpressed in pancreatic cancer, ovarian cancer, melanoma, and malignant glioma.

Patent literature (CN114014941A) discloses that IL13Rα2 is a receptor polypeptide for IL-13, is expressed almost exclusively in cancer cells, and is not expressed in normal tissue cells except for the testis.

The first IL13Rα2-specific CAR-T clinical trial was completed in 2015, recruiting three patients with recurrent glioblastoma (GBM). Overall, the treatment was well tolerated in these patients, with only transient encephalitis. It was reported that one patient showed decreased IL13Rα2 expression in the tumor following CAR-T treatment, indicating that the treatment promoted tumor regression.

### SUMMARY

The present disclosure adopts phage display technology to prepare and screen antibodies or antigen-binding fragments thereof. The screened antibodies or the antigen-binding fragments thereof specifically bind to IL13Rα2 and do not cross-bind to IL13Rα1. They can effectively kill tumor cells expressing IL13Rα2 and increase the secretion level of cytokines. Specifically:
In a first aspect of the present disclosure, provided is an antibody or an antigen-binding fragment thereof, and the antibody or the antigen-binding fragment thereof comprises a CDR1, a CDR2, and a CDR3, wherein the amino acid sequence of the CDR1 comprises an amino acid sequence set forth in GX₁X₂FSX₃AW (SEQ ID NO: 1) or GTIFSDSF (SEQ ID NO: 2), or, comprises an amino acid sequence having 90% or higher homology to an amino acid sequence set forth in GX₁X₂FSX₃AW (SEQ ID NO: 1) or GTIFSDSF (SEQ ID NO: 2);
the amino acid sequence of the CDR2 comprises an amino acid sequence set forth in ITSX₄GX₅TV (SEQ ID NO: 3) or ITSGDITN (SEQ ID NO: 4), or, comprises an amino acid sequence having 90% or higher homology to an amino acid sequence set forth in ITSX₄GX₅TV (SEQ ID NO: 3) or ITSGDITN (SEQ ID NO: 4);
the amino acid sequence of the CDR3 comprises an amino acid sequence set forth in NARPGAVNSY (SEQ ID NO: 5), NSRIYTRSY (SEQ ID NO: 6), or NARPRGLSFSSY (SEQ ID NO: 7), or, comprises an amino acid sequence having 90% or higher homology to an amino acid sequence set forth in NARPGAVNSY (SEQ ID NO: 5), NSRIYTRSY (SEQ ID NO: 6), or NARPRGLSFSSY (SEQ ID NO: 7).

Preferably, the amino acid sequence of the CDR1 is set forth in GX₁X₂FSX₃AW (SEQ ID NO: 1) or GTIFSDSF (SEQ ID NO: 2).

Preferably, the amino acid sequence of the CDR2 is set forth in ITSX₄GX₅TV (SEQ ID NO: 3) or ITSGDITN (SEQ ID NO: 4).

Preferably, the amino acid sequence of the CDR3 is set forth in NARPGAVNSY (SEQ ID NO: 5), NSRIYTRSY (SEQ ID NO: 6), or NARPRGLSFSSY (SEQ ID NO: 7).

X₁, X₂, X₃, X₄, and X₅ are each independently selected from a natural amino acid and an unnatural amino acid.

X₁ is selected from, but not limited to, I, S, F, T, and V; preferably, X₁ is selected from F and I.

X₂ is selected from, but not limited to, I, T, and A; preferably, X₂ is selected from T and I.

X₃ is selected from, but not limited to, G, N, S, V, L, A, D, Y, and I; preferably, X₃ is selected from S and G.

X₄ is selected from, but not limited to, G, A, S, D, and T; preferably, X₄ is selected from S and G.

X₅ is selected from, but not limited to, S, D, I, T, V, and M; preferably, X₅ is selected from D and S.

In one specific embodiment of the present disclosure, the amino acid sequence of the CDR1 comprises an amino acid sequence set forth in GFTFSSAW (SEQ ID NO: 15), GTIFSDSF (SEQ ID NO: 2), or GIIFSGAW (SEQ ID NO: 17), or, comprises an amino acid sequence having 90% or higher homology to an amino acid sequence set forth in GFTFSSAW (SEQ ID NO: 15), GTIFSDSF (SEQ ID NO: 2), or GIIFSGAW (SEQ ID NO: 17).

The amino acid sequence of the CDR2 comprises an amino acid sequence set forth in ITSSGDTV (SEQ ID NO: 18), ITSGGSTV (SEQ ID NO: 19), or ITSGDITN (SEQ ID NO: 4), or, comprises an amino acid sequence having 90% or higher homology to an amino acid sequence set forth in ITSSGDTV (SEQ ID NO: 18), ITSGGSTV (SEQ ID NO: 19), or ITSGDITN (SEQ ID NO: 4).

The amino acid sequence of the CDR3 comprises an amino acid sequence set forth in NARPGAVNSY (SEQ ID NO: 5), NSRIYTRSY (SEQ ID NO: 6), or NARPRGLSFSSY (SEQ ID NO: 7), or, comprises an amino acid sequence having 90% or higher homology to an amino acid sequence set forth in NARPGAVNSY (SEQ ID NO: 5), NSRIYTRSY (SEQ ID NO: 6), or NARPRGLSFSSY (SEQ ID NO: 7).

Preferably, the antibody or the antigen-binding fragment thereof comprises any one of the following groups:
A) the CDR1 comprising GFTFSSAW (SEQ ID NO: 15), or, comprising an amino acid sequence having 90% or higher homology to the amino acid sequence set forth in GFTFSSAW (SEQ ID NO: 15); the CDR2 comprising ITSSGDTV (SEQ ID NO: 18), or, comprising an amino acid sequence having 90% or higher homology to the amino acid sequence set forth in ITSSGDTV (SEQ ID NO: 18); the CDR3 comprising NARPGAVNSY (SEQ ID NO: 5), or, comprising an amino acid sequence having 90% or higher homology to the amino acid sequence set forth in NARPGAVNSY (SEQ ID NO: 5);
B) the CDR1 comprising GTIFSDSF (SEQ ID NO: 2), or, comprising an amino acid sequence having 90% or higher homology to the amino acid sequence set forth in GTIFSDSF (SEQ ID NO: 2); the CDR2 comprising ITSGGSTV (SEQ ID NO: 19), or, comprising an amino acid sequence having 90% or higher homology to the amino acid sequence set forth in ITSGGSTV (SEQ ID NO: 19); the CDR3 comprising NSRIYTRSY (SEQ ID NO: 6), or, comprising an amino acid sequence having 90% or higher homology to the amino acid sequence set forth in NSRIYTRSY (SEQ ID NO: 6); or,
C) the CDR1 comprising GIIFSGAW (SEQ ID NO: 17), or, comprising an amino acid sequence having 90% or higher homology to the amino acid sequence set forth in GIIFSGAW (SEQ ID NO: 17); the CDR2 comprising ITSGDITN (SEQ ID NO: 4), or, comprising an amino acid sequence having 90% or higher homology to the amino acid sequence set forth in ITSGDITN (SEQ ID NO: 4); the CDR3 comprising NARPRGLSFSSY (SEQ ID NO: 7), or, comprising an amino acid sequence having 90% or higher homology to the amino acid sequence set forth in NARPRGLSFSSY (SEQ ID NO: 7).

In one specific embodiment of the present disclosure, the antibody or the antigen-binding fragment thereof comprises a CDR1, a CDR2, and a CDR3, wherein the CDR1 is set forth in GFTFSSAW (SEQ ID NO: 15), the CDR2 is set forth in ITSSGDTV (SEQ ID NO: 18), and the CDR3 is set forth in NARPGAVNSY (SEQ ID NO: 5).

In one specific embodiment of the present disclosure, the antibody or the antigen-binding fragment thereof comprises a CDR1, a CDR2, and a CDR3, wherein the CDR1 is set forth in GTIFSDSF (SEQ ID NO: 2), the CDR2 is set forth in ITSGGSTV (SEQ ID NO: 19), and the CDR3 is set forth in NSRIYTRSY (SEQ ID NO: 6).

In one specific embodiment of the present disclosure, the antibody or the antigen-binding fragment thereof comprises a CDR1, a CDR2, and a CDR3, wherein the CDR1 is set forth in GIIFSGAW (SEQ ID NO: 17), the CDR2 is set forth in ITSGDITN (SEQ ID NO: 4), and the CDR3 is set forth in NARPRGLSFSSY (SEQ ID NO: 7).

Preferably, in the variable region of the antibody, the arrangement in sequential order is the CDR1, the CDR2, and the CDR3. A framework region may also be comprised between the CDR1 and the CDR2, and/or a framework region may also be comprised between the CDR2 and the CDR3. Preferably, the antibody or the antigen-binding fragment thereof includes, but is not limited to, a Fab, an Fd, a Fab', a Fab'-SH, an Fv, an scFv, a F(ab')2, a single domain antibody, a diabody (dAb), a full-length antibody, or a linear antibody.

In one specific embodiment of the present disclosure, the antibody or the antigen-binding fragment thereof is a single domain antibody.

The single domain antibody comprises one heavy chain variable region (VHH) and two conventional CH2 and CH3 regions.

Preferably, the amino acid sequence of the antibody or the antigen-binding fragment thereof comprises an amino acid sequence set forth in any one of SEQ ID NOs: 8-9 or 16 or comprises an amino acid sequence having 90% or higher homology to an amino acid sequence set forth in any one of SEQ ID NOs: 8-9 or 16.

Further preferably, the amino acid sequence of the antibody or the antigen-binding fragment thereof is set forth in any one of SEQ ID NOs: 8-9 or 16.

Preferably, the antibody or the antigen-binding fragment thereof binds to an IL13Rα2 protein.

Preferably, the IL13Rα2 protein includes a human or non-human animal IL13Rα2 protein.

The non-human animal is preferably a rodent. More preferably, the rodent includes, but is not limited to, a rat or a mouse.

In a second aspect of the present disclosure, provided is a nucleic acid encoding the CDR1, the CDR2, and the CDR3 of the present disclosure.

In a third aspect of the present disclosure, provided is a nucleic acid, and the nucleic acid encodes the antibody or the antigen-binding fragment thereof according to any one of the above in the first aspect.

Preferably, the nucleotide sequence of the nucleic acid comprises a nucleotide sequence set forth in any one of SEQ ID NOs: 10-12 or comprises a nucleotide sequence having 90% or higher homology to a nucleotide sequence set forth in any one of SEQ ID NOs: 10-12.

In one specific embodiment of the present disclosure, the nucleotide sequence of the nucleic acid is set forth in any one of SEQ ID NOs: 10-12.

In a fourth aspect of the present disclosure, provided is an expression vector, and the expression vector comprises the nucleic acid according to any one of the above.

Preferably, the expression vector is a eukaryotic expression vector or a prokaryotic expression vector.

Further preferably, the prokaryotic expression vector is selected from an *Escherichia coli* expression system series vector, a *Bacillus subtilis* expression system series vector, and a streptomycin expression system series vector.

Further preferably, the eukaryotic expression vector is selected from a yeast expression system series vector, a fungal expression system series vector, an insect cell expression system series vector, and a mammalian cell expression system series vector.

In one specific embodiment of the present disclosure, the expression vector is a eukaryotic expression vector.

In a fifth aspect of the present disclosure, provided is a host cell.

Preferably, the host cell comprises the nucleic acid according to any one of the above, and/or, the expression vector according to any one of the above.

Preferably, the host cell may be a eukaryotic cell or a prokaryotic cell.

In a sixth aspect of the present disclosure, provided is a chimeric antigen receptor, and the chimeric antigen receptor comprises an extracellular antigen-binding domain, wherein the extracellular antigen-binding domain comprises the antibody or the antigen-binding fragment thereof according to the first aspect described above.

Preferably, the chimeric antigen receptor further comprises an intracellular domain, a transmembrane domain, and/or a hinge region.

Preferably, the hinge region links the extracellular antigen-binding domain to the transmembrane domain.

Preferably, the transmembrane domain is selected from one of or a combination of two or more of a CD3ζ polypeptide, a CD4 polypeptide, a CD8 polypeptide, a CD28 polypeptide, a CD28-41BB polypeptide, an OX40 polypeptide, an ICOS polypeptide, a CTLA-4 polypeptide, a PD-1 polypeptide, an LAG-3 polypeptide, a 2B4 polypeptide, and a BTLA polypeptide.

Preferably, the intracellular domain comprises a signaling domain, preferably CD3ζ.

Further preferably, the intracellular domain further comprises a co-stimulatory domain.

Preferably, the intracellular domain is selected from one of or a combination of two or more of CD28, ICOS, 4-1BB, OX-40, CD27, and CD3ζ. Further preferably, the intracellular domain is 4-1BB-CD3ζ.

Preferably, the hinge region is selected from an extracellular hinge region of CD8, CD8α, CD28, or IgG, preferably CD8.

In one specific embodiment of the present disclosure, the structure of the chimeric antigen receptor is: the antibody or the antigen-binding fragment thereof described above-CD8HT-4-1BB-CD3ζ.

In a seventh aspect of the present disclosure, provided is a construction method for the chimeric antigen receptor according to the sixth aspect described above, and the construction method comprises:
A) obtaining a nucleic acid encoding the antibody or the antigen-binding fragment thereof according to the first aspect described above;
B) transforming the nucleic acid obtained in step A) into a host cell, and inducing the expression thereof.

Preferably, the nucleic acid is the nucleic acid according to the third aspect of the present disclosure.

In an eighth aspect of the present disclosure, provided is an immune cell, and the immune cell expresses the antibody or the antigen-binding fragment thereof according to the first aspect described above and/or the chimeric antigen receptor according to the sixth aspect described above.

Preferably, the immune cell is obtained by transfecting an immune cell with the nucleic acid according to any one of the above.

Preferably, the immune cell includes, but is not limited to, one of or two or more of a lymphocyte (including a T cell, a B cell, and an NK cell), a dendritic cell, a monocyte/macrophage, a granulocyte, or a mast cell.

In one specific embodiment of the present disclosure, the immune cell is a CAR-T cell.

In a ninth aspect of the present disclosure, provided is an immunoconjugate.

The immunoconjugate comprises the antibody or the antigen-binding fragment thereof according to the first aspect or the chimeric antigen receptor according to the sixth aspect described above conjugated to a therapeutic agent or a diagnostic reagent.

In a tenth aspect of the present disclosure, provided is use of the antibody or the antigen-binding fragment thereof, the nucleic acid, the expression vector, the host cell, the chimeric antigen receptor, or the immune cell described in the present disclosure in preparing an antibody-drug conjugate or a multifunctional antibody.

In an eleventh aspect of the present disclosure, provided is a medicament or a pharmaceutical composition, and the medicament or the pharmaceutical composition comprises one of or two or more of the antibody or the antigen-binding fragment thereof, the nucleic acid, the expression vector, the host cell, the chimeric antigen receptor, or the immune cell.

The medicament or the pharmaceutical composition specifically targets cells of IL13Rα2.

Preferably, the medicament or the pharmaceutical composition further comprises a pharmaceutically acceptable auxiliary material.

Preferably, the pharmaceutically acceptable auxiliary material is selected from one of or a combination of two or more of a carrier, an excipient, a diluent, a lubricant, a wetting agent, an emulsifier, a preservative, an antioxidant, a buffer, a bacteriostat, a solute that renders a formulation isotonic with the blood of a recipient, a suspension, a suspending agent, a solubilizer, a thickener, a stabilizer, a sweetening agent, and a spice.

Preferably, the formulation of the medicament or the pharmaceutical composition may be in the form of a syrup, an elixir, a suspension, a powder, a granule, a tablet, a capsule, a lozenge, an aqueous solution, a cream, an ointment, a lotion, a gel, or an emulsion. The various dosage forms of the medicament can be prepared according to conventional production methods in the field of pharmaceuticals.

Preferably, the pharmaceutical formulation is preferably a formulation in unit dosage.

The amount of the pharmaceutically active ingredients of the formulation in unit dosage may be varied or adjusted from 0.001 mg to 1000 mg, depending on the specific application and the potency of the pharmaceutically active ingredients.

According to specific needs, the pharmaceutical composition may further comprise an additional suitable therapeutic agent.

Formulations of the medicament or the pharmaceutical composition suitable for parenteral administration, for example, administration by intravenous, intramuscular, intradermal, and subcutaneous routes, include aqueous and non-aqueous isotonic sterile injection solutions, which may include antioxidants, buffers, bacteriostats, and solutes that render the formulation isotonic with the blood of the recipient; and aqueous and non-aqueous sterile suspensions, which may include suspending agents, solubilizers, thickeners, stabilizers, or preservatives. In the practice of the present disclosure, medicaments or pharmaceutical compositions may be administered by, for example, intravenous infusion, as well as oral, topical, intraperitoneal, intravesical, and intrathecal administration. Formulations may be presented in unit-dose or multi-dose sealed containers, such as ampoules and vials. Solutions for injection and suspensions can be prepared from sterile powders, granules, and tablets of the type previously described.

Preferably, the medicament or the pharmaceutical composition may be used alone or in combination with an additional therapeutic agent.

In a twelfth aspect of the present disclosure, provided is a diagnostic kit, and the diagnostic kit comprises one of or two or more of the antibody or the antigen-binding fragment thereof, the nucleic acid, the expression vector, the host cell, the chimeric antigen receptor, or the immune cell.

The diagnostic kit is for diagnosing a disease associated with IL13Rα2 expression.

Preferably, the disease associated with IL13Rα2 expression includes a disease where inhibition of IL13Rα2 expression provides a therapeutic benefit.

Preferably, the disease associated with IL13Rα2 expression includes a disease where IL13Rα2 is expressed, highly expressed, or overexpressed.

Preferably, the disease associated with IL13Rα2 expression includes, but is not limited to, dermatitis, a pulmonary disease, an intestinal disease, a tumor, and the like.

Further preferably, the dermatitis includes, but is not limited to, atopic dermatitis.

Further preferably, the pulmonary disease includes, but is not limited to, pulmonary fibrosis (preferably idiopathic pulmonary fibrosis) or a lung tumor.

Further preferably, the intestinal disease includes colitis (preferably dextran sulfate sodium-induced colitis) or an intestinal tumor.

Further preferably, the tumor includes, but is not limited to, lymphoma, lung cancer, cervical cancer, leukemia, ovarian cancer, nasopharyngeal cancer, breast cancer, endometrial cancer, colon cancer, liver cancer, brain cancer, rectal cancer, gastric cancer, bladder cancer, glioma (e.g., brain glioma), lung cancer, bronchial cancer, bone cancer, prostate cancer, pancreatic cancer, liver and bile duct cancer, esophageal cancer, renal cancer, thyroid cancer, head and neck cancer, testicular cancer, glioblastoma, astrocytoma, melanoma, myelodysplastic syndrome, and sarcoma, wherein the leukemia is selected from one of or two or more of acute lymphocytic leukemia, acute myelogenous leukemia, myeloid leukemia, chronic lymphocytic leukemia, multiple myeloma, plasma cell leukemia, and chronic myelogenous leukemia.

More preferably, the lymphoma includes Hodgkin's lymphoma and non-Hodgkin's lymphoma, including one of or two or more of B-cell lymphoma, diffuse large B-cell lymphoma, follicular lymphoma, mantle cell lymphoma, marginal zone B-cell lymphoma, T-cell lymphoma, or Waldenström macroglobulinemia.

More preferably, the sarcoma includes one of or two or more of osteosarcoma, Ewing's sarcoma, leiomyosarcoma, synovial sarcoma, soft tissue sarcoma, angiosarcoma, liposarcoma, fibrosarcoma, rhabdomyosarcoma, or chondrosarcoma.

In one specific embodiment of the present disclosure, the tumor is selected from, but is not limited to, one of or two or more of glioma, a brain tumor, ovarian cancer, liver cancer, malignant mesothelioma, renal cancer, thyroid cancer, epidermoid carcinoma, Kaposi's sarcoma, prostate cancer, pancreatic cancer, breast cancer, or melanoma, wherein
the glioma includes, but is not limited to, glioblastoma and brain glioma;
the renal cancer includes renal cell carcinoma and clear cell renal cell carcinoma;
the Kaposi's sarcoma, prostate cancer, or pancreatic cancer is preferably AIDS-related Kaposi's sarcoma, prostate cancer, or pancreatic cancer.

In one specific embodiment of the present disclosure, the tumor includes melanoma and glioma. In a thirteenth aspect of the present disclosure, provided is use of the antibody or the antigen-binding fragment thereof, the nucleic acid, the expression vector, the host cell, the chimeric antigen receptor, or the immune cell.

Preferably, the use includes use in preparing a product for diagnosing and/or treating a disease associated with IL13Rα2 expression.

Further preferably, the disease associated with IL13Rα2 expression includes a disease where inhibition of IL13Rα2 expression provides a therapeutic benefit.

Further preferably, the disease associated with IL13Rα2 expression includes a disease where IL13Rα2 is expressed, highly expressed, or overexpressed.

Preferably, the product includes, but is not limited to, a diagnostic kit, a medicament, or a pharmaceutical composition.

Preferably, the disease associated with IL13Rα2 expression includes, but is not limited to, dermatitis, a pulmonary disease, an intestinal disease, a tumor, and the like.

Further preferably, the dermatitis includes, but is not limited to, atopic dermatitis.

Further preferably, the pulmonary disease includes, but is not limited to, pulmonary fibrosis (preferably idiopathic pulmonary fibrosis) or a lung tumor.

Further preferably, the intestinal disease includes colitis (preferably dextran sulfate sodium-induced colitis) or an intestinal tumor.

Further preferably, the tumor includes, but is not limited to, lymphoma, lung cancer, cervical cancer, leukemia, ovarian cancer, nasopharyngeal cancer, breast cancer, endometrial cancer, colon cancer, liver cancer, brain cancer, rectal cancer, gastric cancer, bladder cancer, glioma, lung cancer, bronchial cancer, bone cancer, prostate cancer, pancreatic cancer, liver and bile duct cancer, esophageal cancer, renal cancer, thyroid cancer, head and neck cancer, testicular cancer, glioblastoma, astrocytoma, melanoma, myelodysplastic syndrome, and sarcoma, wherein the leukemia is selected from one of or two or more of acute lymphocytic leukemia, acute myelogenous leukemia, myeloid leukemia, chronic lymphocytic leukemia, multiple myeloma, plasma cell leukemia, and chronic myelogenous leukemia.

More preferably, the lymphoma includes Hodgkin's lymphoma and non-Hodgkin's lymphoma, including one of or two or more of B-cell lymphoma, diffuse large B-cell lymphoma, follicular lymphoma, mantle cell lymphoma, marginal zone B-cell lymphoma, T-cell lymphoma, or Waldenström macroglobulinemia.

More preferably, the sarcoma includes one of or two or more of osteosarcoma, Ewing's sarcoma, leiomyosarcoma, synovial sarcoma, soft tissue sarcoma, angiosarcoma, liposarcoma, fibrosarcoma, rhabdomyosarcoma, or chondrosarcoma.

In one specific embodiment of the present disclosure, the tumor is selected from, but is not limited to, one of or two or more of glioma, a brain tumor, ovarian cancer, liver cancer, malignant mesothelioma, renal cancer, thyroid cancer, epidermoid carcinoma, Kaposi's sarcoma, prostate cancer, pancreatic cancer, breast cancer, or melanoma, wherein
the glioma includes, but is not limited to, glioblastoma and brain glioma;
the renal cancer includes renal cell carcinoma and clear cell renal cell carcinoma;
the Kaposi's sarcoma, prostate cancer, or pancreatic cancer is preferably AIDS-related Kaposi's sarcoma, prostate cancer, or pancreatic cancer.

In one specific embodiment of the present disclosure, the tumor includes melanoma and glioma. In a fourteenth aspect of the present disclosure, provided is a method for treating a disease associated with IL13Rα2 expression.

Preferably, the method comprises administering to a subject in need the antibody or the antigen-binding fragment thereof described in the present disclosure, the chimeric antigen receptor described in the present disclosure, or the immune cell described in the present disclosure.

In one specific embodiment of the present disclosure, the immune cell is a CAR-T cell.

Preferably, the treatment of the disease associated with IL13Rα2 expression comprises the use of CAR-T cells at 1.0E+05 cells/mL to 1.0E+08 cells/mL, such as 1.0E+05 cells/mL, 3.0E+05 cells/mL, 5.0E+05 cells/mL, 6.0E+05 cells/mL, 7.0E+05 cells/mL, 8.0E+05 cells/mL, 9.0E+05 cells/mL, 1.0E+06 cells/mL, 3.0E+06 cells/mL, 4.0E+06 cells/mL, 5.0E+06 cells/mL, 6.0E+06 cells/mL, 7.0E+06 cells/mL, 8.0E+06 cells/mL, 9.0E+06 cells/mL, 1.0E+07 cells/mL, 2.0E+07 cells/mL, 3.0E+07 cells/mL, 4.0E+07 cells/mL, 5.0E+07 cells/mL, 6.0E+07 cells/mL, 7.0E+07 cells/mL, 8.0E+07 cells/mL, 9.0E+07 cells/mL, or 1.0E+08 cells/mL.

Preferably, the treatment of the disease associated with IL13Rα2 expression comprises the use of CAR-T cells, and further preferably, CAR-T cells:target cells is greater than or equal to 1/10, 1/5, 1/3, 1/2, 1, 2, 3, 4, 5, 6, 7, 8, 9, 10, or the like.

In a fifteenth aspect of the present disclosure, provided is a method for detecting IL13Rα2, and the method comprises contacting the antibody or the antigen-binding fragment thereof described above, the chimeric antigen receptor described above, or the immune cell described above with a sample to be detected.

The sample to be detected includes, but is not limited to, a body fluid, a cell, a tissue, or an organ. Preferably, the sample to be detected is blood, serum, plasma, or a tumor cell.

Preferably, the method further comprises adding a labeling substance; further preferably, the labeling substance includes, but is not limited to, a radioactive label, a fluorescent label, a reporter cell, or the like.

In one specific embodiment of the present disclosure, the method comprises contacting a sample to be detected with a detectable amount of the antibody or the antigen-binding fragment thereof described above, the chimeric antigen receptor described above, or the immune cell described above, and adding a labeling substance.

Preferably, the detectable amount is at least 0.1 µg/mL, such as 0.1 µg/mL, 0.2 µg/mL, 0.3 µg/mL, 0.4 µg/mL, 0.5 µg/mL, 0.6 µg/mL, 0.7 µg/mL, 0.8 µg/mL, 0.9 µg/mL, 1 µg/mL, 2 µg/mL, 3 µg/mL, 4 µg/mL, 5 µg/mL, or more.

The term "comprise", "comprising", or "include" described in the present disclosure is an openended description that includes the specified ingredients or steps as described, as well as other specified ingredients or steps that do not substantially affect the technical effect. When the term is used to describe a sequence of a protein or nucleic acid, the protein or nucleic acid may consist of the sequence, or may have additional amino acids or nucleotides at one or two ends of the protein or nucleic acid, while retaining the same or similar activity as the original sequence.

The term "and/or" in the present disclosure encompasses all combinations of the items connected by the term, and each combination should be deemed to have been individually listed herein. For example, "A and/or B" encompasses "A", "A and B", and "B". For another example, "A, B, and/or C" encompasses "A", "B", "C", "A and B", "A and C", "B and C", and "A and B and C". The term "treating" or "treatment" described in the present disclosure refers to slowing, interrupting, arresting, controlling, stopping, reducing, or reversing the progression or severity of a sign, symptom, disorder, condition, or disease after the disease has begun to progress, but it does not necessarily involve the complete elimination of all disease-related signs, symptoms, conditions, or disorders.

The term "diagnosing" in the present disclosure refers to the determination of whether a patient has suffered from, is suffering from, or will suffer from a disease or condition in the past, at the time of diagnosis, or in the future, or the determination of the progression or likely progression in the future of a disease.

The "pharmaceutically acceptable" described in the present disclosure refers to the biological activity and properties of neither significantly stimulating an organism nor inhibiting the active substance of the administered product.

The "antigen-binding fragment" described in the present disclosure is a portion of an antibody that retains the specific binding activity of the antibody, i.e., any portion of the antibody that is capable of specifically binding to an epitope on a target molecule of the antibody. It includes, for example, a Fab, an Fv, an Fd, a Fab', a Fab'-SH, a F(ab')2, and variants of these fragments, such as the heavy chain and/or light chain of the antibody, the heavy chain variable region and/or light chain variable region of the antibody, or a single or 2 or more CDRs from the heavy or light chains of the antibody. Among these, Fab is a monovalent fragment consisting of VL, VH, CL, and CH1 domains. F(ab')2 is a bivalent fragment comprising two Fab fragments linked by a disulfide bridge at the hinge region. Fd is an Fd fragment consisting of VH and CH1 domains. Fv is an Fv fragment consisting of VL and VH domains of a single arm of an antibody. Fab' is a Fab fragment with one or more cysteine residues at the C terminus of the CH1 domain. Fab'-SH is a Fab' in which the cysteine residue(s) of the constant domain bear at least one free thiol group. Single domain antibodies refer to a class of antibodies lacking an antibody light chain and having only a heavy chain, and are also referred to as nanobodies due to their small molecular weight. VH represents a heavy chain variable region, VL represents a light chain variable region, CL represents a light chain, and CH1 is CH1 constituting a heavy chain constant region.

The "unnatural amino acid" described in the present disclosure is an amino- and carboxyl-containing compound that is not naturally found in proteins. Preferably, the unnatural amino acid is any unnatural amino acid known in the art. Further preferably, the unnatural amino acid includes, but is not limited to N-ethylaspartyl, hydroxylysine, 3-hydroxyproline, 2-aminobutyric acid, β-alanine, β-aminopropionic acid, 2-aminoadipic acid, 3-aminoadipic acid, 4-aminobutyric acid, 6-aminohexanoic acid, 2-aminoheptanoic acid, allo-isoleucine, isodesmosine, 4-hydroxyproline, allo-hydroxylysine, 2-aminoisobutyric acid, N-methylglycine, N-methylisoleucine, 3-aminoisobutyric acid, 6-N-methyllysine, 2,4-diaminobutyric acid, N-methylvaline, ornithine, norleucine, norvaline, desmosine, 2,2'-diaminopimelic acid, 2,3-diaminopropionic acid, N-ethylglycine, 2-aminopimelic acid, or the like. Certainly, the "unnatural amino acid" in the present application may also be a modified derivative of a natural amino acid.

The term "CDR" described in the present disclosure refers to a complementarity determining region within an antibody variable sequence. For each variable region, there are three CDRs in each variable region of the heavy and/or light chains, which are referred to as CDR1, CDR2, and CDR3. The exact boundaries of these CDRs are defined differently according to different systems. The systems include the Kabat, Chothia, IMGT, AbM, or Contact system, and the like, and the CDRs herein may be defined according to any one of these systems.

### BRIEF DESCRIPTION OF THE DRAWINGS

Hereinafter, examples of the present disclosure will be described in detail with reference to the drawings, where:
FIG. 1 shows the detection results of the purified antibodies;
FIG. 2 shows the mean fluorescence intensities of the six groups of 1-E9, 1-E10, 2-G01, 1-C10, 10-C4, and 7-G8;
FIG. 3 shows the mean fluorescence intensities of the three groups of 4-C2, 4-F10, and 7-F2;
FIG. 4 shows the killing effects of the single domain antibodies screened in the present disclosure on 293T, 293T-IL13Rα2, A375, and U251 cells, where T represents T cells, G01 represents CAR-T cells prepared from the 2-G01 single domain antibody, C2 represents CAR-T cells prepared from the 4-C2 single domain antibody, F10 represents CAR-T cells prepared from the 4-F10 single domain antibody, and EQ represents a positive control;
FIG. 5 shows the effects of the single domain antibodies screened in the present disclosure on the IFN-γ cytokine release concentration in 293T, A375, and U251 cells, where T represents T cells, G01 represents CAR-T cells prepared from the 2-G01 single domain antibody, C2 represents CAR-T cells prepared from the 4-C2 single domain antibody, F10 represents CAR-T cells prepared from the 4-F10 single domain antibody, EQ represents a positive control, Alone represents spontaneous release from effector cells, and the histograms for each group are, from left to right, T, EQ, G01, C2, and F10;
FIG. 6 shows the effects of the single domain antibodies screened in the present disclosure on the IL-2 cytokine release concentration in 293T, A375, and U251 cells, where T represents T cells, G01 represents CAR-T cells prepared from the 2-G01 single domain antibody, C2 represents CAR-T cells prepared from the 4-C2 single domain antibody, F10 represents CAR-T cells prepared from the 4-F10 single domain antibody, EQ represents a positive control, Alone represents spontaneous release from effector cells, and the histograms for each group are, from left to right, T, EQ, G01, C2, and F10;
FIG. 7 shows the effect of the single domain antibody screened in the present disclosure on tumor volume, where G01 represents CAR-T cells prepared from the 2-G01 single domain antibody;
FIG. 8 shows the mean tumor volumes in mice of treatment groups with different doses after subcutaneous injection of A375, where G01 represents CAR-T cells prepared from the 2-G01 single domain antibody, and **** represents that p < 0.0001;
FIG. 9 shows the body weight of tumor-bearing mice in different treatment groups, G01 representing CAR-T cells prepared from the 2-G01 single domain antibody;
FIG. 10 shows the concentration optimization of the 2-G01 antibody for screening;
FIG. 11 shows the concentration optimization of the 4-F10 antibody for screening;
FIG. 12 shows the screening results of the membrane protein screening array of the 2-G01 antibody;
FIG. 13 shows the screening results of the membrane protein screening array of the 4-F10 antibody;
FIG. 14 shows the binding indices of the 2-G01 antibody at different concentrations to the top 20 targets ranked by binding indices;
FIG. 15 shows the binding indices of the 4-F10 antibody at different concentrations to the top 20 targets ranked by binding indices.

### DETAILED DESCRIPTION

The technical solutions in the examples of the present disclosure will be described clearly and completely below with reference to the drawings. It is obvious that the described examples are only a part of the examples of the present disclosure, rather than all of them. Based on the examples of the present disclosure, all other examples obtained by those of ordinary skill in the art without inventive efforts shall fall within the protection scope of the present disclosure.

Some methods used in the examples of the present disclosure are as follows:
Preparation method for CAR-T cells:
1. Cell preparation: PBMCs isolated from peripheral blood are activated with CD3/CD28 magnetic beads, and lentivirus infection is performed 24 h later.
2. Lentivirus infection: according to the experimental design, an appropriate amount of activated T cells is taken and centrifuged at 300 g at room temperature for 5 min, and the supernatant is discarded. The T cells are resuspended in a medium, and subsequently, an appropriate amount of concentrated lentivirus solution (the amount of virus was determined according to the difference in the virus titer and MOI) is added. The mixture is gently pipetted, and the final cell concentration is 2.0E+10⁶/mL. The cells are incubated in an incubator with 5% CO₂ overnight for infection.
3. The medium is refreshed 24 h after the lentivirus infection, and CAR T cells are continuously cultured for expansion. The number of cells and the efficiency of cell infection are observed.
4. The cell killing assay is performed 10 days after the cells were infected with the lentiviruses.

### Example 1: Phage Display Library Screening for Development of IL13Rα2 Single Domain Antibodies

1. After an alpaca was immunized, the immune potency was detected by ELISA. The results are shown in Table 1 below. After a certain potency was reached, peripheral blood was drawn from the alpaca for PBMC cell isolation, and an antibody phage library was constructed.
2. Panning of phage display library:
An IL13Rα2 recombinant protein was used for panning. The phage display library was then incubated with the recombinant protein. The recombinant phages bound to the target antigen were eluted using TEA and amplified. After 3-4 rounds of panning, single clones were selected for sequencing.

**Table 1**

| Serum dilution ratio | Preimmune serum | | Serum after third immunization | | Serum after fourth immunization | |
|---|---|---|---|---|---|---|
| 1:1K | 0.208 | 0.197 | 2.387 | 2.513 | 3.412 | 3.325 |
| 1:2K | 0.132 | 0.114 | 1.434 | 1.508 | 2.410 | 2.464 |
| 1:4K | 0.098 | 0.091 | 0.904 | 0.978 | 1.706 | 1.712 |
| 1:8K | 0.081 | 0.076 | 0.556 | 0.578 | 1.125 | 1.071 |
| 1:16K | 0.082 | 0.077 | 0.348 | 0.348 | 0.665 | 0.689 |
| 1:32K | 0.070 | 0.068 | 0.213 | 0.226 | 0.409 | 0.420 |
| 1:64K | 0.076 | 0.069 | 0.153 | 0.158 | 0.272 | 0.286 |
| PBS | 0.067 | 0.070 | 0.069 | 0.060 | 0.061 | 0.068 |

The target antigen recombinant protein was co-incubated with the single domain antibody phage display library to enrich phages specifically binding to the target antigen. After four rounds of enrichment, the input and output for each round were counted, and the enrichment factor for each round was calculated. The results are shown in Table 2 below.

**Table 2**

| Round | Input (PFU) | Output (PFU) | Enrichment factor |
|---|---|---|---|
| First round | 3.63E+12 | 8.50E+06 | 4.27E+05 |
| Second round | 4.37E+12 | 1.18E+07 | 3.72E+05 |
| Third round | 2.31E+12 | 1.9E+06 | 1.22E+06 |
| Fourth round | 2.43E+12 | 1.29E+06 | 1.88E+06 |

### 3. Expression and purification of candidate single domain antibodies

The bacterial solution of the target clones was taken for PCR amplification. The PCR products were transiently transfected into CHO-K1 cells. The expressed antibody supernatant was collected and detected by fluorescence-activated cell sorting (FACS).

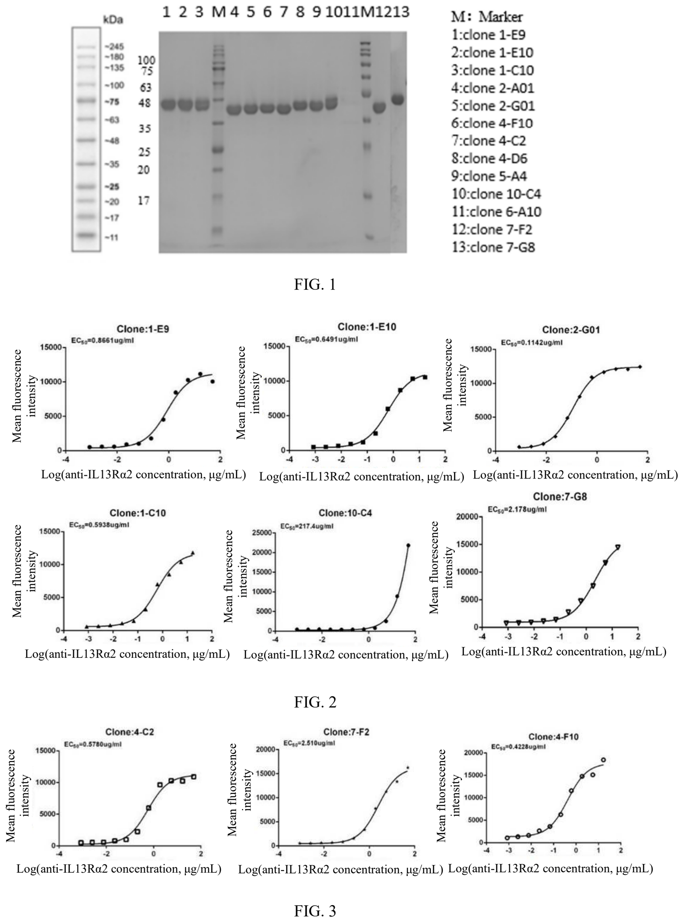

13 distinct clones (1-E9, 1-E10, 1-C10, 2-A01, 2-G01, 4-F10, 4-C2, 4-D6, 5-A4, 10-C4, 6-A10, 7-F2, and 7-G8) that specifically bound to IL13Rα2 were selected for the construction of eukaryotic expression vectors. The detection results of the purified antibodies are shown in FIG. 1. Since 6-A10 showed no band, it was excluded. The remaining 12 clones were used as candidate single domain antibodies.

### 4. FACS detection of candidate single domain antibodies with gradient dilution

CHO-K1/IL13Rα2 cells were used for detection. The purified candidate single domain antibodies were added. After setting an appropriate initial concentration, the 3-fold gradient dilution was performed to obtain 11 points. Detection was performed by FACS using a secondary antibody. Exemplary mean fluorescence intensities are shown in FIGs. 2-3. Exemplary EC50 (µg/mL) values for each antibody are listed in Table 3 below.

**Table 3**

| Antibody number | 1-E9 | 1-E10 | 1-C10 | 2-G01 | 4-F10 | 4-C2 | 10-C4 | 7-F2 | 7-G8 |
|---|---|---|---|---|---|---|---|---|---|
| EC50 | 0.8661 | 0.6491 | 0.5938 | 0.1142 | 0.4228 | 0.5780 | 217.4 | 2.510 | 2.178 |

Result analysis: The above candidate single domain antibodies all bound to CHO-K1/IL13Rα2 cells. Except for the candidate antibody 10-C4, the other candidate antibodies all exhibited strong binding to CHO-K1/IL13Rα2 cells.

### 5. FACS detection of candidate single domain antibodies with IL13Rα1 transiently transfected cells

The results showed that the above candidate single domain antibodies did not cross-bind to 293F/IL13Rα1 cells.

Furthermore, after analysis and verification of the above sequences, the following three key sequence antibodies were subjected to further study: 2-G01, 4-F10, and 4-C2. Their sequences are provided below, with the EQ antibody used as a positive control, the sequence of which is also provided below.
Nucleotide sequence encoding 2-G01: 
CAGGTACAGCTGAAAGAGTCTGGGGGAGGCTTGGTGCAGGCTGGGGGGTCTCTGA
GACTCTCCTGTGTAGCCTCTGGATTCACATTCAGTAGCGCGTGGATGGGCTGGTAC
CGCCAGGCTGCAGGGAAGCAGCGCGAATTGTTCGCATCTATTACTAGTAGTGGTGA
CACAGTCTATGCAGGCTCCGTGAAGGGCCGATTCTCCATCTCTAAGAACAACGCCG
AGAACACTCTTTATCTGCAAATGGATGCGCTGAGGCCTGAAGATACAGCCGTCTAT
TACTGCAATGCCCGTCCGGGGGCGGTCAATAGTTACTGGGGCCAGGGGACCCAGGT
CACCGTCTCCACA (SEQ ID NO: 10);
Nucleotide sequence encoding 4-C2: 
CAGTTGCGGCTCTTGGAGTCTGGGGGAGGCCTGGTGCAGGCTGGGGAGTCTCTGAG
ACTCTCCTGTGCTGTCTCTGGAACCATCTTTAGTGACAGTTTCCTGGGCTGGTACCG
CCAGGCTCCAGGGAAGCAGCGCGAGTTGATCGCAAGTATTACTAGTGGTGGTAGC
ACAGTTTATGCAGGGTCCGTAAAGGGCCGATTCACCATCTCCAGAGACAACGCCCA
GGACACGGTGTATCTGCAAATGAACAGCCTGAAACCTGAGGACACAGCCGTCTATT
ACTGTAATTCACGCATCTATACTCGATCCTACTGGGGCCAGGGGACCCAAGTCACC
GTCTCTTCT (SEQ ID NO: 11);
Nucleotide sequence encoding 4-F10: CAGGTGCAGCTGGTAGAGTCTGGGGGCGGCTTGGTGCAGGCTGGGGGGTCTCTGA 
GACTCTCCTGTGCAGCCTCTGGAATCATCTTCAGTGGCGCGTGGATGGGCTGGTAC
CGCCAGGCTGCAGGCGAGCAGCGCGAGTTGGTCGCATCTATTACTAGTGGTGATAT
CACAAACTATGCAGACTCCGTGAAGGGCCGATTCACCATCTCTAGAGACAACGCCG
AAAACACGGGCTATCTGCAAATGAATACGCTGAAGCCTGAGGATACAGCCGTCTAT
TACTGTAACGCCCGTCCTAGGGGTTTAAGTTTCAGTTCTTACTGGGGCCAGGGGAC
CCTGGTCACCGTCTCCTCA (SEQ ID NO: 12);
Nucleic acid sequence encoding EQ antibody: 
GGCCCCGTGCCCCCTAGCACCGCCCTGCGCTACCTGATCGAGGAACTGGTGAACAT
CACCCAGAACCAGAAAGCCCCCCTGTGCAACGGCAGCATGGTGTGGAGCATCAAC
CTGACCGCCGGCATGTACTGTGCCGCCCTGGAAAGCCTGATCAACGTGAGCGGCTG
CAGCGCCATCGAGAAAACCCAGCGGATGCTGTCCGGCTTCTGCCCCCACAAGGTGT
CCGCCGGACAGTTCAGCAGCCTGCACGTGCGGGACACCAAGATCGAGGTGGCCCA
GTTCGTGAAGGACCTGCTGCTGCACCTGAAGAAGCTGTTCCGGGAGGGCCGGTTCA
AC (SEQ ID NO: 13);
Amino acid sequence of 2-G01: 
QVQLKESGGGLVQAGGSLRLSCVASGFTFSSAWMGWYRQAAGKQRELFASITSSGDT
VYAGSVKGRFSISKNNAENTLYLQMDALRPEDTAVYYCNARPGAVNSYWGQGTQVT
VST (SEQ ID NO: 16);
Amino acid sequence of 4-C2: 
QLRLLESGGGLVQAGESLRLSCAVSGTIFSDSFLGWYRQAPGKQRELIASITSGGSTVY
AGSVKGRFTISRDNAQDTVYLQMNSLKPEDTAVYYCNSRIYTRSYWGQGTQVTVSS
(SEQ ID NO: 8);
Amino acid sequence of 4-F10: 
QVQLVESGGGLVQAGGSLRLSCAASGIIFSGAWMGWYRQAAGEQRELVASITSGDITN
YADSVKGRFTISRDNAENTGYLQMNTLKPEDTAVYYCNARPRGLSFSSYWGQGTLVT
VSS (SEQ ID NO: 9);
Amino acid sequence of EQ antibody:

GPVPPSTALRYLIEELVNITQNQKAPLCNGSMVWSINLTAGMYCAALESLINVSGCSAIE
KTQRMLSGFCPHKVSAGQFSSLHVRDTKIEVAQFVKDLLLHLKKLFREGRFN (SEQ ID
NO: 14).

### Example 2: Validation of Antibody Effectiveness by In Vitro Cell Killing Experiment

1. Target cells 293T, 293T-IL13Rα2, A375 (human malignant melanoma cells), and U251 (human glioma cells) were counted separately, collected, and centrifuged at 300 g at room temperature for 5 min, and the supernatant was discarded.
2. The cells were resuspended in a medium separately and centrifuged at 300 g at room temperature for 5 min.
3. The supernatant was discarded, and step 2 was repeated.
4. The cells were resuspended in an appropriate amount of medium, and the cell density was counted.
5. Preparation of CAR-T cells: The CAR structure was an antibody sequence (the antibody sequence was 2-G01, 4-C2, or 4-F10 screened in Example 1)-CD8HT-4-1BB-CD3ζ structure. CAR-T cells were prepared using the method described above.
6. CAR-T cells were taken and centrifuged at 300 g for 5 min, and the supernatant was discarded.
7. The cells were resuspended in an appropriate amount of medium separately, sampled for counting, and centrifuged at 300 g at room temperature for 5 min.
8. The supernatant was discarded, and step 7 was repeated.
9. The CAR-T cells were resuspended in PBS, and the cell density was counted.

Exemplary information for CAR-T cells prepared using the 2-G01 antibody is shown in Table 4 below:

**Table 4**

| Name | Cell density | Volume | Viability | Total cell count | Freezing density |
|---|---|---|---|---|---|
| T | 4.72E+06 | 50 | 90.49% | 2.36E+08 | 2.00E+07 |
| G01CAR-T | 7.71E+06 | 50 | 91.91% | 3.84E+08 | 2.86E+07 |

A 96-well cell culture plate (purchased from: Corning, Cat. No.: 3599) was taken, and CAR-T/NC cells and target cells were co-incubated. The effector-to-target ratios of effective CAR-T/NC cells to the number of target cells for each well were 5:1, 3:1, 1:1, and 1:2, with the total volume not exceeding 100 µL.

10. The cells were cultured and co-incubated at 37 °C for detection.

11. The cell killing efficiency was detected.

12. The experimental data were analyzed, and the experimental results and conclusions were presented graphically.

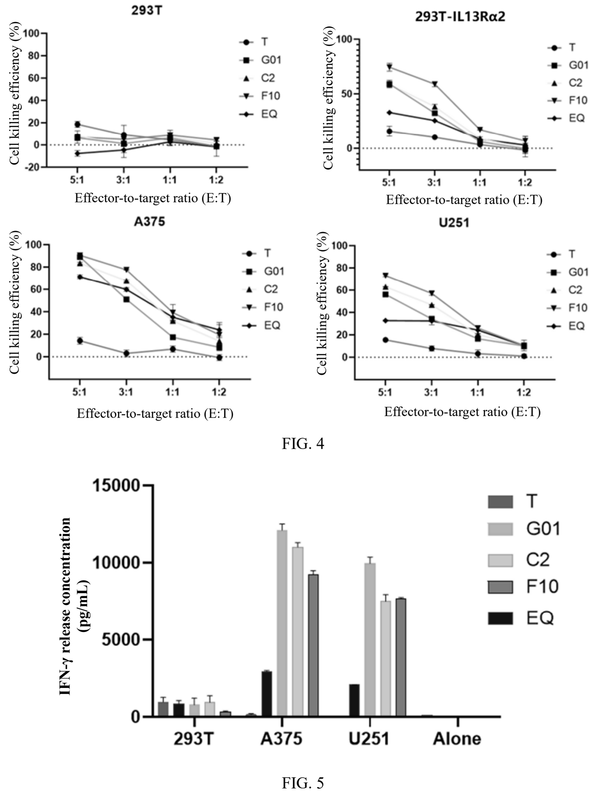

The results are shown in FIG. 4. The three single domain antibodies described in the present disclosure had no killing effect on 293T cells, but had a killing effect on 293T cells expressing IL13Rα2, A375 cells, and U251 cells. Particularly, when the effector-to-target ratio (E:T) was 5:1, the killing effect was the strongest.

### Example 3: Cytokine Detection

1. Target cells 293T, A375, and U251 were counted separately, collected, and centrifuged at 300 g at room temperature for 5 min, and the supernatant was discarded.
2. The cells were resuspended in a medium separately and centrifuged at 300 g at room temperature for 5 min.
3. The supernatant was discarded, and step 2 was repeated.
4. The cells were resuspended in an appropriate amount of medium, and the cell density was counted.
5. CAR-T cells were taken and centrifuged at 300 g for 5 min, and the supernatant was discarded (the preparation of CAR-T cells was the same as step 5 in Example 2).
6. The cells were resuspended in an appropriate amount of medium separately, sampled for counting, and centrifuged at 300 g at room temperature for 5 min.
7. The supernatant was discarded, and step 6 was repeated.
8. The CAR-T cells were resuspended in PBS, and the cell density was counted.
   A 96-well cell culture plate was taken, and CAR-T/NC cells and target cells were co-incubated: the effector-to-target ratio of effective CAR-T/NC cells to the number of target cells for each well was 1:1, with the total volume not exceeding 200 µL.
9. After culturing and co-incubation at 37 °C, the cell supernatant was collected and detected by ELISA, and the data were analyzed to obtain results.

The results are shown in FIGs. 5-6. The use of the single domain antibodies described in the present application significantly increased the release concentrations of cytokines IFN-γ and IL-2.

### Example 4: Antitumor Effect of IL-13Rα2 VHH CAR T Cells in A375 Syngeneic Mouse Melanoma Model

Cell culture: A375 cells (CL-0014, Procell) were rapidly thawed in a water bath at 37 °C. The cells were resuspended in RPMI-1640 (purchased from: Gibco, Cat. No.: C11875500BT) containing 10% fetal bovine serum (purchased from: ExCell Bio, Cat. No.: FSP500) and a 100 µg/mL penicillin-streptomycin solution (purchased from: Procell, Cat. No.: PB180120, containing 100 µg/mL penicillin and 100 µg/mL streptomycin). The cells were placed in an incubator (purchased from: Thermo Scientific, Cat. No.: 371) at 37 °C with 5% CO₂ and passaged once every 2 days. A375 cells were seeded into 150 mm culture dishes (purchased from: NEST, Cat. No.: 715001). The cells were collected when the cell quantity reached 2 × 10⁷ cells/dish.

Preparation of CAR-T cell reinfusion liquid: Frozen T cells and CAR-T cells prepared based on the 2-G01 antibody (hereinafter referred to as G01 CAR-T cells) were retrieved from liquid nitrogen and thawed in a water bath at 37 °C. The cells were transferred to a centrifuge tube containing PBS and centrifuged (centrifuge purchased from: Eppendorf, Cat. No.: 5804R). The supernatant was removed, and the cells were resuspended in PBS. Cell counting was performed (using a cell counter, purchased from: Invitrogen, Cat. No.: Countess II). The concentration of T cells was adjusted to 5.0E+07 cells/mL. The effective cell concentration of G01 CAR-T cells was adjusted to 1.0E+06 cells/mL, 3.0E+06 cells/mL, and 1.0E+07 cells/mL.

Construction of A375 tumor mouse model: Tumor cells A375 were subcutaneously inoculated into 30 NCG mice (purchased from: Jiangsu GemPharmatech Co., Ltd., animal production license number: SCXK (Jiangsu) 2018-0008) at 2 × 10⁶ cells per mouse. 4 days after inoculation, mice with relatively uniform tumor volume (about 30 mm³) were selected and randomly divided into 5 groups, and the grouping is shown in Table 5. Group A1 (n = 6) received a tail vein reinfusion of 100 µL of PBS per mouse as the solvent control group; group A2 (n = 6) received a tail vein reinfusion of 100 µL of 5.0E+07 cells/mL T cells per mouse; group A3 (n = 6) received a tail vein reinfusion of 100 µL of 1.0E+06 cells/mL effective G01 CAR-T cells per mouse; group A4 (n = 6) received a tail vein reinfusion of 100 µL of 3.0E+06 cells/mL G01 CAR-T cells per mouse; group A5 (n = 6) received a tail vein reinfusion of 100 µL of 1.0E+07 cells/mL G01 CAR-T cells per mouse. Tumor volume size was measured twice a week using a vernier caliper. The tumor volume calculation formula was as follows: tumor volume = (length of long axis of tumor) × (length of short axis of tumor)²/2. The tumor inhibition rate was calculated as follows: tumor inhibition rate = (mean tumor volume of treatment group - mean tumor volume of control group)/mean tumor volume of control group × 100%. Statistical comparison of tumor growth was analyzed by two-way ANOVA. Additionally, the body weight of the mice in each group was measured twice a week.

**Table 5**

| **Group** | **Number of mice (mice)** | **Administration dose and mode** |
|---|---|---|
| A1 | 6 | PBS, 100 µL, tail vein reinfusion |
| A2 | 6 | T cells (5.0E+07 cells/mL), 100 µL, tail vein reinfusion |
| A3 | 6 | G01 CAR-T cells (1.0E+06 cells/mL), 100 µL, tail vein reinfusion |
| A4 | 6 | G01 CAR-T cells (3.0E+06 cells/mL), 100 µL, tail vein reinfusion |
| A5 | 6 | G01 CAR-T cells (1.0E+07 cells/mL), 100 µL, tail vein reinfusion |

Data analysis: The data were statistically analyzed using Graph pad 10. The experimental data were expressed as Mean ± SD, and the data were tested using the F-test and two-sample variance analysis. When p < 0.05, a two-sample t-test assuming unequal variances was used for calculation. When p > 0.05, a t-test assuming equal variances was used for calculation (test standard α = 0.05, two-sided).

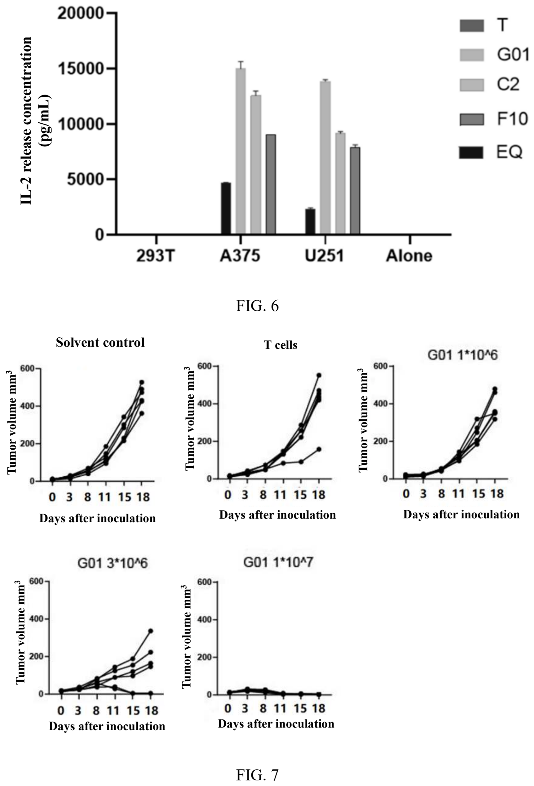

Experimental results: Compared with the control group, the reinfusion of G01 CAR-T cells in groups A4 and A5 significantly inhibited tumor growth. The tumor inhibition rate in group A2 was 14.80%, in group A3 was 8.36%, in group A4 was 67.59%, and in group A5 was 99.89%. In group A4, 14 days after the reinfusion of G01 CAR-T cells, tumors in 2 mice almost disappeared, and the tumor volume growth in the remaining 4 mice was slow; compared with group A2, tumor growth was significantly inhibited. In group A5, 14 days after the reinfusion of G01 CAR-T cells, tumors in 6 mice almost completely disappeared; compared with group A2, tumor growth was significantly inhibited, with an inhibition rate close to 100% (see FIGs. 7-8).

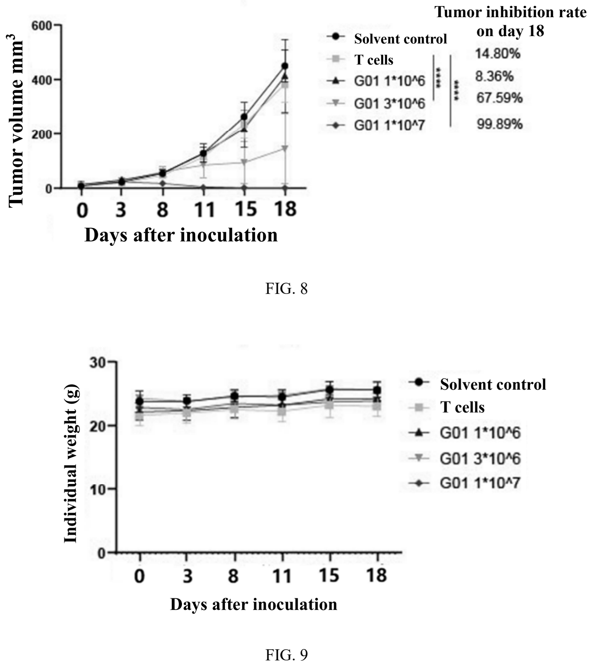

Additionally, the body weight of the mice in the 5 groups was detected, and no significant difference was found among the 5 groups (see FIG. 9), indicating that the use of the CAR-T cells described in the present application is safe.

In summary, IL-13Rα2 CAR-T cells had a significant tumor inhibition effect on the A735 melanoma model mice. A significant tumor inhibition effect was observed when the effective cell reinfusion amount reached 3.0E+06 cells/mouse. When the effective cell reinfusion amount reached 1.0E+07 cells/mouse, the tumor inhibition rate was close to 100%. Furthermore, there was no impact on the individual body weight of the mice.

### Example 5: Validation of Binding Specificity of Antibodies to IL-13Rα2 by Membrane Protein Screening Array

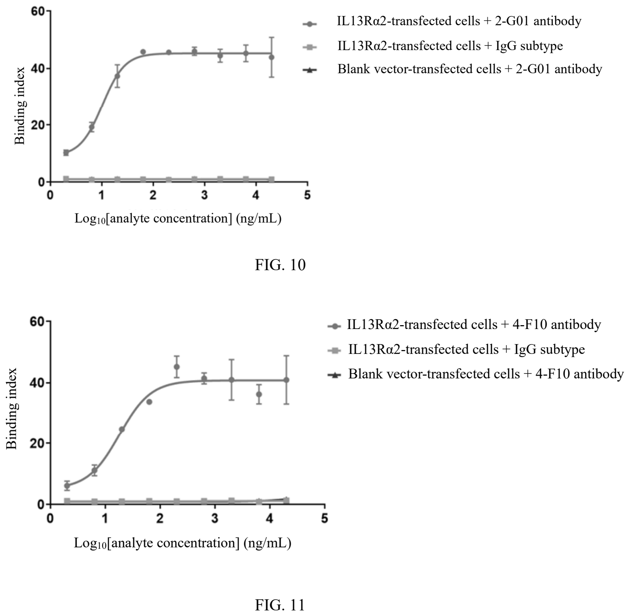

The membrane protein screening array AB5000 was used to examine the binding specificity of the antibodies obtained from the screening in Example 1 to 4032 human membrane proteins (receptors, transporters, enzymes, etc.). The procedures were as follows:
1) Experimental setup. Determination of optimal test sample concentration for screening.
   To determine the optimal concentration of the samples for the target screening phase, target cells were transfected with vectors encoding the gene for the sample's binding target (positive) or with an empty vector (negative control) in a 96-well plate, respectively. After transfection, each sample was subjected to a 3.16-fold gradient dilution starting from a maximum concentration of 20 µg/mL, resulting in a total of 9 detection concentrations. The sample dilutions were added to a culture plate along with reporter cells in the same number as the target cells, followed by co-incubation. Fluorescence signals were acquired using a fluorescence microplate reader. The optimal screening concentration for the test samples was determined via the binding index to achieve low background and high sensitivity. The optimal screening concentration was determined to be 0.1 µg/mL (FIGs. 10-11) and was used for subsequent experiments.
2) Membrane protein screening array: The test samples were screened using the membrane protein screening array AB5000 to identify binding targets.

To identify the binding targets, 4032 human membrane proteins were expressed individually in HEK-293T cells arranged in separate wells of a 96-well plate. After culturing, the test samples and reporter cells were added to the transfected cells. The optimal screening concentration for the test samples was 0.1 µg/mL. Fluorescence signals were acquired using a fluorescence microplate reader. The resulting binding values were subsequently normalized and transformed to give individual numerical values (i.e., the binding index) representing the binding of the test samples relative to each target protein.

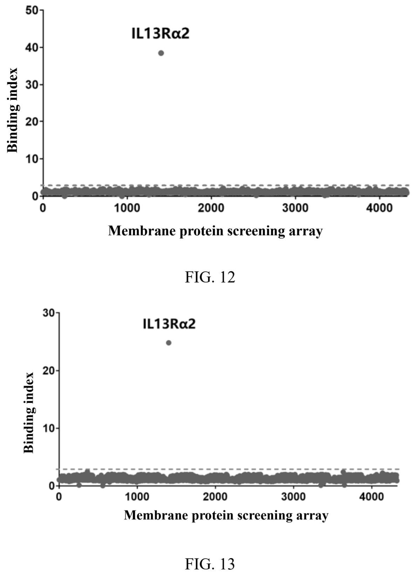

The membrane protein screening array results for the 2-G01 antibody and the 4-F10 antibody are shown in FIGs. 12-13.

Exemplarily, taking the top 20 targets ranked by binding index as an example, the target information and binding indices are listed below:
2-G01 antibody: IL13Rα2 (UniPort ID: Q14627, binding index: 38.49), CACNG3 (UniPort ID: 060359, binding index: 2.10), GOLM2 (UniPort ID: Q6P4E1, binding index: 2.10), CSF1R (UniPort ID: P07333, binding index: 2.08), RIGI (UniPort ID: 095786, binding index: 2.03), ICOS (UniPort ID: Q9Y6W8, binding index: 2.02), GDPD5 (UniPort ID: Q8WTR4, binding index: 2.02), IGSF5 (UniPort ID: Q9NSI5, binding index: 2.01), GFRA1 (UniPort ID: P56159, binding index: 2.00), MUSK (UniPort ID: 015146, binding index: 1.99), TNFSF9 (UniPort ID: P41273, binding index: 1.98), GPM6A (UniPort ID: P51674, binding index: 1.97), FGFR4 (UniPort ID: P22455, binding index: 1.96), ASGR1 (UniPort ID: P07306, binding index: 1.96), ILDR1 (UniPort ID: Q86SU0, binding index: 1.95), ROR1 (UniPort ID: Q01973, binding index: 1.95), CALCRL (UniPort ID: Q16602, binding index: 1.94), OR2B3 (UniPort ID: 076000, binding index: 1.92), SMO (UniPort ID: Q99835, binding index: 1.92), and RNF19A (UniPort ID: Q9NV58, binding index: 1.91).

4-F10 antibody: IL13Rα2 (UniPort ID: Q14627, binding index: 24.82), DDX58 (UniPort ID: 095786, binding index: 2.48), GPM6A (UniPort ID: P51674, binding index: 2.4), OR4A47 (UniPort ID: Q6IF82, binding index: 2.22), CLEC10A (UniPort ID: Q8IUN9, binding index: 2.15), C2CD2L (UniPort ID: 014523, binding index: 2.03), SELL (UniPort ID: P14151, binding index: 1.99), OR1D5 (UniPort ID: P58170, binding index: 1.95), GPR33 (UniPort ID: Q49SQ1, binding index: 1.93), CDHR5 (UniPort ID: Q9HBB8, binding index: 1.93), ANKK1 (UniPort ID: Q8NFD2, binding index: 1.91), CPNE7 (UniPort ID: Q9UBL6, binding index: 1.90), KCNH4 (UniPort ID: Q9UQ05, binding index: 1.89), FAM189A2 (UniPort ID: Q15884, binding index: 1.89), DPY19L3 (UniPort ID: Q6ZPD9, binding index: 1.86), PRIMA1 (UniPort ID: Q86XR5, binding index: 1.83), CYYR1 (UniPort ID: Q96J86, binding index: 1.82), PTPRK (UniPort ID: Q15262, binding index: 1.82), DIRAS1 (UniPort ID: O95057, binding index: 1.81), and CHIC1 (UniPort ID: Q5VXU3, binding index: 1.76).

### (3) Target validation. Validation of newly identified targets using reporter cells.

To determine the optimal concentration of the samples for the target screening phase, target cells were transfected with vectors encoding the gene for the sample's binding target (positive) or with an empty vector (negative control) in a 96-well plate, respectively. After transfection, each sample was subjected to a 3.16-fold gradient dilution starting from a maximum concentration of 20 µg/mL, resulting in a total of 9 detection concentrations. The sample dilutions were added to a culture plate along with reporter cells in the same number as the target cells, followed by co-incubation. Fluorescence signals were acquired using a fluorescence microplate reader.

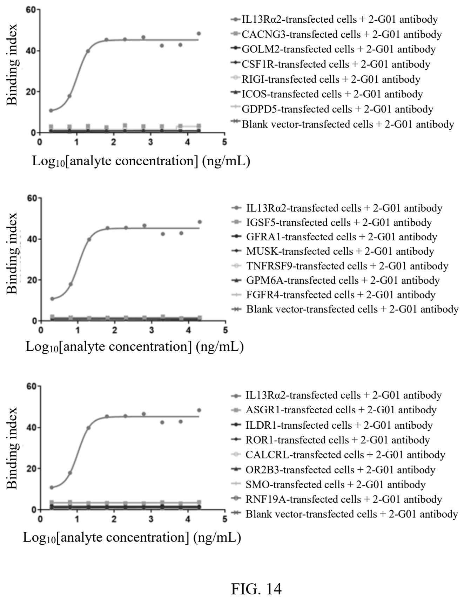

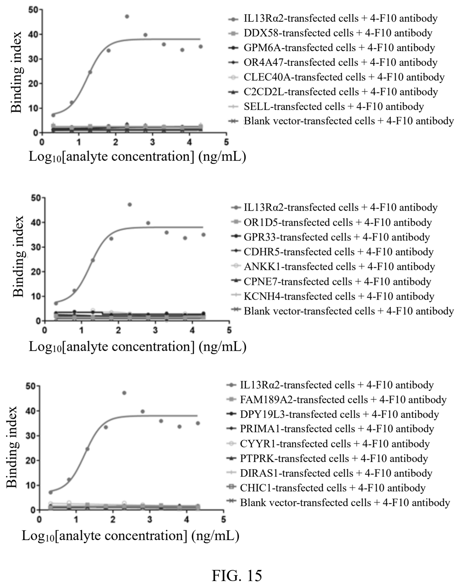

The results are shown in FIGs. 14-15.

In summary, the antibodies screened in the present application specifically bind to IL13Rα2 and can be used for the detection of the IL13Rα2 protein.

The preferred embodiments of the present disclosure are described in detail above. However, the present disclosure is not limited to the specific details in the embodiments described above. Within the scope of the technical concept of the present disclosure, various simple modifications can be made to the technical solutions of the present disclosure, and all such simple modifications will fall within the protection scope of the present disclosure.

In addition, it should be noted that the various specific technical features described in the specific embodiments described above can be combined in any suitable manner provided that no contradiction arises. In order to avoid unnecessary repetition, all possible combinations will not be illustrated separately in the present disclosure.

## Claims

1. An antibody or an antigen-binding fragment thereof, comprising a CDR1, a CDR2, and a CDR3, wherein
an amino acid sequence of the CDR1 comprises an amino acid sequence set forth in GX₁X₂FSX₃AW (SEQ ID NO: 1) or GTIFSDSF (SEQ ID NO: 2), or, comprises an amino acid sequence having 90% or higher homology to an amino acid sequence set forth in GX₁X₂FSX₃AW (SEQ ID NO: 1) or GTIFSDSF (SEQ ID NO: 2);
an amino acid sequence of the CDR2 comprises an amino acid sequence set forth in ITSX₄GX₅TV (SEQ ID NO: 3) or ITSGDITN (SEQ ID NO: 4), or, comprises an amino acid sequence having 90% or higher homology to an amino acid sequence set forth in ITSX₄GX₅TV (SEQ ID NO: 3) or ITSGDITN (SEQ ID NO: 4);
an amino acid sequence of the CDR3 comprises an amino acid sequence set forth in NARPGAVNSY (SEQ ID NO: 5), NSRIYTRSY (SEQ ID NO: 6), or NARPRGLSFSSY (SEQ ID NO: 7), or, comprises an amino acid sequence having 90% or higher homology to an amino acid sequence set forth in NARPGAVNSY (SEQ ID NO: 5), NSRIYTRSY (SEQ ID NO: 6), or NARPRGLSFSSY (SEQ ID NO: 7);
wherein, X₁ is selected from I, S, F, T, and V;
X₂ is selected from I, T, and A;
X₃ is selected from G, N, S, V, L, A, D, Y, and I;
X₄ is selected from G, A, S, D, and T;
X₅ is selected from S, D, I, T, V, and M.

2. The antibody or the antigen-binding fragment thereof according to claim 1, wherein:
X₁ is selected from F and I;
X₂ is selected from T and I;
X₃ is selected from S and G;
X₄ is selected from S and G;
X₅ is selected from D and S.

3. The antibody or the antigen-binding fragment thereof according to claim 1 or 2, wherein an amino acid sequence of the CDR1 comprises an amino acid sequence set forth in GFTFSSAW (SEQ ID NO: 15), GTIFSDSF (SEQ ID NO: 2), or GIIFSGAW (SEQ ID NO: 17), or, comprises an amino acid sequence having 90% or higher homology to an amino acid sequence set forth in GFTFSSAW (SEQ ID NO: 15), GTIFSDSF (SEQ ID NO: 2), or GIIFSGAW (SEQ ID NO: 17); an amino acid sequence of the CDR2 comprises an amino acid sequence set forth in ITSSGDTV (SEQ ID NO: 18), ITSGGSTV (SEQ ID NO: 19), or ITSGDITN (SEQ ID NO: 4), or, comprises an amino acid sequence having 90% or higher homology to an amino acid sequence set forth in ITSSGDTV (SEQ ID NO: 18), ITSGGSTV (SEQ ID NO: 19), or ITSGDITN (SEQ ID NO: 4); an amino acid sequence of the CDR3 comprises an amino acid sequence set forth in NARPGAVNSY (SEQ ID NO: 5), NSRIYTRSY (SEQ ID NO: 6), or NARPRGLSFSSY (SEQ ID NO: 7), or, comprises an amino acid sequence having 90% or higher homology to an amino acid sequence set forth in NARPGAVNSY (SEQ ID NO: 5), NSRIYTRSY (SEQ ID NO: 6), or NARPRGLSFSSY (SEQ ID NO: 7).

4. The antibody or the antigen-binding fragment thereof according to any one of claims 1-3, comprising any one of the following groups:
A) the CDR1 comprising GFTFSSAW (SEQ ID NO: 15), or, comprising an amino acid sequence having 90% or higher homology to the amino acid sequence set forth in GFTFSSAW (SEQ ID NO: 15); the CDR2 comprising ITSSGDTV (SEQ ID NO: 18), or, comprising an amino acid sequence having 90% or higher homology to the amino acid sequence set forth in ITSSGDTV (SEQ ID NO: 18); the CDR3 comprising NARPGAVNSY (SEQ ID NO: 5), or, comprising an amino acid sequence having 90% or higher homology to the amino acid sequence set forth in NARPGAVNSY (SEQ ID NO: 5);
B) the CDR1 comprising GTIFSDSF (SEQ ID NO: 2), or, comprising an amino acid sequence having 90% or higher homology to the amino acid sequence set forth in GTIFSDSF (SEQ ID NO: 2); the CDR2 comprising ITSGGSTV (SEQ ID NO: 19), or, comprising an amino acid sequence having 90% or higher homology to the amino acid sequence set forth in ITSGGSTV (SEQ ID NO: 19); the CDR3 comprising NSRIYTRSY (SEQ ID NO: 6), or, comprising an amino acid sequence having 90% or higher homology to the amino acid sequence set forth in NSRIYTRSY (SEQ ID NO: 6); or,
C) the CDR1 comprising GIIFSGAW (SEQ ID NO: 17), or, comprising an amino acid sequence having 90% or higher homology to the amino acid sequence set forth in GIIFSGAW (SEQ ID NO: 17); the CDR2 comprising ITSGDITN (SEQ ID NO: 4), or, comprising an amino acid sequence having 90% or higher homology to the amino acid sequence set forth in ITSGDITN (SEQ ID NO: 4); the CDR3 comprising NARPRGLSFSSY (SEQ ID NO: 7), or, comprising an amino acid sequence having 90% or higher homology to the amino acid sequence set forth in NARPRGLSFSSY (SEQ ID NO: 7).

5. The antibody or the antigen-binding fragment thereof according to any one of claims 1-4, comprising a Fab, an Fd, a Fab', a Fab'-SH, an Fv, an scFv, a F(ab')2, a single domain antibody, a diabody (dAb), a full-length antibody, or a linear antibody.

6. The antibody or the antigen-binding fragment thereof according to any one of claims 1-5, wherein the antibody or the antigen-binding fragment thereof binds to an IL13Rα2 protein; preferably, the IL13Rα2 protein comprises a human or non-human animal IL13Rα2 protein.

7. The antibody or the antigen-binding fragment thereof according to any one of claims 1-6, wherein an amino acid sequence of the antibody or the antigen-binding fragment thereof comprises an amino acid sequence set forth in any one of SEQ ID NOs: 8-9 or 16 or comprises an amino acid sequence having 90% or higher homology to an amino acid sequence set forth in any one of SEQ ID NOs: 8-9 or 16.

8. A nucleic acid, wherein the nucleic acid encodes the antibody or the antigen-binding fragment thereof according to any one of claims 1-7;
preferably, the nucleic acid comprises a nucleotide sequence set forth in any one of SEQ ID NOs: 10-12 or comprises a nucleotide sequence having 90% or higher homology to a nucleotide sequence set forth in any one of SEQ ID NOs: 10-12.

9. An expression vector or a host cell, comprising the nucleic acid according to claim 8.

10. A chimeric antigen receptor, comprising an extracellular antigen-binding domain, wherein the extracellular antigen-binding domain comprises the antibody or the antigen-binding fragment thereof according to any one of claims 1-7.

11. The chimeric antigen receptor according to claim 10, further comprising an intracellular domain, a transmembrane domain, and/or a hinge region, wherein
preferably, the hinge region links the extracellular antigen-binding domain to the transmembrane domain.

12. The chimeric antigen receptor according to claim 11, wherein the transmembrane domain is selected from one of or a combination of two or more of a CD3ζ polypeptide, a CD4 polypeptide, a CD8 polypeptide, a CD28 polypeptide, a CD28-41BB polypeptide, an OX40 polypeptide, an ICOS polypeptide, a CTLA-4 polypeptide, a PD-1 polypeptide, an LAG-3 polypeptide, a 2B4 polypeptide, and a BTLA polypeptide.

13. The chimeric antigen receptor according to claim 11 or 12, wherein the intracellular domain is selected from one of or a combination of two or more of CD28, ICOS, 4-1BB, OX-40, CD27, and CD3ζ.

14. The chimeric antigen receptor according to any one of claims 11-13, wherein the hinge region is selected from an extracellular hinge region of CD8, CD28, or IgG.

15. An immune cell, wherein the immune cell expresses the antibody or the antigen-binding fragment thereof according to any one of claims 1-7, or, the chimeric antigen receptor according to any one of claims 10-14.

16. The immune cell according to claim 15, wherein the immune cell is selected from one of or two or more of a lymphocyte (e.g., a T cell, a B cell, and an NK cell), a dendritic cell, a monocyte/macrophage, a granulocyte, and a mast cell;
preferably, the immune cell is a CAR-T cell.

17. A medicament or a diagnostic kit, comprising one of or two or more of the antibody or the antigen-binding fragment thereof according to any one of claims 1-7, the nucleic acid according to claim 8, the expression vector or the host cell according to claim 9, the chimeric antigen receptor according to any one of claims 10-14, or the immune cell according to any one of claims 15-16.

18. Use of the antibody or the antigen-binding fragment thereof according to any one of claims 1-7, the nucleic acid according to claim 8, the expression vector or the host cell according to claim 9, the chimeric antigen receptor according to any one of claims 10-14, or the immune cell according to any one of claims 15-16, comprising use in preparing a product for diagnosing and/or treating a disease associated with IL13Rα2 expression, wherein
preferably, the disease associated with IL13Rα2 expression comprises a disease where inhibition of IL13Rα2 expression provides a therapeutic benefit.

19. The use according to claim 18, wherein the disease associated with IL13Rα2 expression is a tumor;
preferably, the tumor is selected from lymphoma, lung cancer, cervical cancer, leukemia, ovarian cancer, nasopharyngeal cancer, breast cancer, endometrial cancer, colon cancer, liver cancer, brain cancer, rectal cancer, gastric cancer, bladder cancer, glioma, lung cancer, bronchial cancer, bone cancer, prostate cancer, pancreatic cancer, liver and bile duct cancer, esophageal cancer, renal cancer, thyroid cancer, head and neck cancer, testicular cancer, glioblastoma, astrocytoma, melanoma, myelodysplastic syndrome, and sarcoma, wherein the leukemia is selected from one of or two or more of acute lymphocytic leukemia, acute myelogenous leukemia, myeloid leukemia, chronic lymphocytic leukemia, multiple myeloma, plasma cell leukemia, and chronic myelogenous leukemia.

20. A method for detecting IL13Rα2, comprising contacting the antibody or the antigen-binding fragment thereof according to any one of claims 1-7, the chimeric antigen receptor according to any one of claims 10-14, or the immune cell according to any one of claims 15-16 with a sample to be detected.
